(19) Europäisches Patentamt
European Patent Office
Office européen des brevets

(11) **EP 1 959 054 A1**

(12) **EUROPEAN PATENT APPLICATION**
published in accordance with Art. 158(3) EPC

(43) Date of publication:
**20.08.2008 Bulletin 2008/34**

(51) Int Cl.:
*D21F 7/06* (2006.01)          *G01N 21/89* (2006.01)

(21) Application number: **06799649.6**

(22) Date of filing: **17.08.2006**

(86) International application number:
**PCT/RU2006/000436**

(87) International publication number:
**WO 2007/021222 (22.02.2007 Gazette 2007/08)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **19.08.2005 RU 2005126376**

(71) Applicants:
• **Velikotny, Mikhail Aleksandrovich**
  **St. Petersburg 195-274 (RU)**
• **Semin, Sergei Jurievich**
  **St. Petersburg 191-025 (RU)**
• **Yakovlev, Sergei Vadimovich**
  **St. Petersburg 191-119 (RU)**

(72) Inventors:
• **Velikotny, Mikhail Aleksandrovich**
  **St. Petersburg 195-274 (RU)**
• **Semin, Sergei Jurievich**
  **St. Petersburg 191-025 (RU)**
• **Yakovlev, Sergei Vadimovich**
  **St. Petersburg 191-119 (RU)**

(74) Representative: **Cheyne, John Robert Alexander M.**
  **HASELTINE LAKE**
  **Redcliff Quay**
  **120 Redcliff Street**
  **Bristol BS1 6HU (GB)**

(54) **OPTICAL ELECTRONIC SYSTEM FOR SETTING UP AND DIAGNOSTICATING A TECHNICAL CONDITION OF PAPER-MAKING MACHINES**

(57)     The invention relates to the pulp-and-paper industry. The aim of said invention is to increase the fidelity of obtainable measuring information, to provide the addressed diagnosis of paper-making machines and to extend operating capabilities of the system by means of programming tools for the computer analysis of a signal and form used thereby for representing results of measurements. The technical result is attained by the use of a light-emitting diode or a semiconductor laser in the form of a radiation source. The inventive system is provided with a photodetector. An preamplifier is connected to the photodetector output, the preamplifier output is connected to the signal input of a digital potentiometer which is connected in series through a bandwidth filter, amplifier, amplitude detector and a low-pass filter to a reference amplifier whose output is connected to a controller analog input and to the input of an analog-to-digital converter. The controller output is connected to the control input of the digital potentiometer and the output of the analog-to-digital converter is connected to a personal computer via an interface.

FIG. 1

**Description**

BACKGROUND OF THE INVENTION

FIELD OF THE INVENTION

The invention is intended for pulp and paper industry and can be used for adjustment and diagnostics of technical condition of paper-making machines.

DESCRIPTION OF RELATED ART

It is well known that the degree of permanency of thickness and uniformity of the structure over the paper web, and particularly the degree of uniformity of distribution of fibres, is the most important characteristic of quality of paper. The said non-uniformity can be observed visually when looking at a paper specimen in the transmitted light. In this case the term "transilluminating paper" is used. Generally the matter concerns the nature of non-uniformity of optical density over the paper web area, the thickness and optical spectrum of transmittance of which may lie beyond the possibilities of visual assessment.

Optical density non-uniformity of paper web depends on several factors associated both with characteristics of the paper pulp and adjustment and parameters of technical condition of the papermaking machine. Among the factors are:

- degree and type of grinding fibrous pulp,
- consistency of concentration of paper pulp, which is fed to the mesh of the papermaking machine,
- variation of the speed of feeding the pulp to the mesh relative to the speed of the mesh itself,
- violation of geometric parameters and the nature of movement of the shafts of the papermaking machine.

Hence average statistical (weighted) parameters that correspond to the spatiotemporal distribution of optical density non-uniformity over the area of the moving paper web reflect not only its quality but also contain concealed information on the influence of process factors. Using known methods of technical diagnostics makes it possible to reveal, expel or optimize the influence of the above-mentioned factors by adjustment of the papermaking machine thus improving quality of paper produced.

The application of optoelectronic methods makes it possible to conduct photometric measurements of paper in order to quantitatively assess its non-uniformity as a paper quality index, which is the result of combined influence of all components of the production process.

Known for the determination of structural non-uniformity of a moving paper web is a device (see USSR Author's Certificate N 896132, class D21F 7/06, published in 1982), which contains a paper web movement velocity meter, a paper web non-uniformity sensor, which comprises a light source and a light detector, a light detector signal analyzer, which consists of a paper web non-uniformity random signal stationary component filter in series with an extractor of an autocorrelation function of this component, a unit for obtaining a correlation window of the autocorrelation function, and a multiplier.

The trouble with this device is that its optical system fails to provide reliable information on structural non-uniformity of the moving paper web. It is caused by the fact that the use of the constant intensity light source does not prevent external background flashes from influencing the information signal.

The use of a radiation source with a wide spectral range results in the fact that not only structure of paper (density non-uniformity) but also distribution of moisture in the paper material is analyzed simultaneously as the source spectrum includes spectral fields of water absorption.

Another drawback of the analog is the influence of a substantial error caused by fluctuations of the position of the surface of the moving paper web, which is caused by the change of distance between the light detector and the surface of the paper web in the scanning zone.

Another known device for continuous control of structural properties of the moving paper web (see USSR Author's Certificate N 195868, class D21f, published in 1967) is chosen as the closest analog (a prototype) for the invention claimed.

This device contains an illuminator (a light source including an incandescent bulb) with a focusing optical system, a light detector, a pre-amplifier, a travel gear for the light detector and the illuminator, an amplifier, a rectifier with a limiter, a plotter, a control unit, a frequency analyzer including a narrow-band tunable filter, a chart strip drive, an analyzer frequency tuning drive, and a holder.

The prototype has the same drawbacks as the analog. Besides, the use of a micro-lens in the illuminator does not permit any fluctuation in the position of the paper web plane. This is caused by a considerable angular aperture of the micro-lens, which gives no way of obtaining the necessary depth resolution of the picture of the septum, and hence - inadmissible defocusing in case of fluctuation in the position of the paper web plane surfaces. This results in random and substantial changes of the size of the scanning light spot in the process of measuring, which appreciably affect reliability of measuring information.

BRIEF SUMMARY OF THE INVENTION

The purpose of this invention is to solve the problem of increasing reliability of measuring information obtained, to provide address diagnostics of papermaking machines, and, additionally, to enhance functionality of the system by the software of the computer-aided analysis of the signal and the appropriate form of representation of the results of measurements.

With this aim in mind, in the first embodiment of the optoelectronic system for adjustment and diagnostics of the technical condition of papermaking machines, comprising a light source with a focusing optical system, which form an illuminator unit, a light detecting unit with a light detector, a pre-amplifier, a travel gear, and a holder, it is proposed according to the invention to use a LED or a photo generator as the light source, the LED or photo generator being powered from a carrier-frequency generator that provides appropriate modulation of radiation thereof, to provide the light detecting unit with a light filter and a condenser arranged in-series in the direction of propagation of the beams on the same optical axis with the illuminator unit, the light detector being located in a focal plane of the condenser; to connect the pre-amplifier to the output of the light detector and the output of the pre-amplifier to a signal input of a digital potentiometer, which is series-connected via a band-pass filter, an amplifier, an amplitude detector and a low-pass filter to a conditioning amplifier, an output of which is connected to an analog input of a controller and to an input of an analog-digital converter. The output of the controller is connected to a control input of the digital potentiometer, and an output of the analog-digital converter is connected via an interface to a personal computer, the diameter of an entrance pupil of the condenser being calculated by the formula:

$$D = \frac{L_{max}}{f'} \bullet d + c'$$

where:

D - the diameter of the entrance pupil of the condenser,
$L_{max}$ - the maximal permissible distance of the paper web from the entrance pupil of the condenser,
f' - the focal distance of the condenser,
d - the diameter of the light sensitive surface of the light detector,
c' - the maximal diameter of the scanning spot of the paper web.

To obtain the technical result in the second embodiment of the optoelectronic system for adjustment and diagnostics of technical condition of the papermaking machines, comprising a light source with a focusing optical system, which form an illuminator unit, a light detecting unit, a pre-amplifier, a travel gear and a holder, it is proposed in accordance with the invention to use as the light source a LED or a photo generator powered from a carrier-frequency generator that provides appropriate modulation of radiation thereof. The light detecting unit of the system is provided with a light filter and a condenser arranged in-series in the direction of propagation of the beams on the same optical axis with the illuminator unit, the light detector being located in a focal plane of the condenser; an output of the light detector is connected to the pre-amplifier, an output of which is connected to a signal input of a digital potentiometer series-connected via a band-pass filter, an amplifier, an amplitude detector and a low-pass filter to a conditioning amplifier, whose output is connected to an analog input of a controller and to an input of an analog differential line driver; an output of the controller is connected to a control input of the digital potentiometer, and an output of the analog differential line driver is connected via a differential line receiver to an input of an analog-digital converter, an output of which is connected via an interface to a personal computer, the diameter of an entrance pupil of the condenser being calculated by the formula:

$$D = \frac{L_{max}}{f'} \bullet d + c'$$

where:

D - the diameter of the entrance pupil of the condenser,
$L_{max}$ - the maximal permissible distance of the paper web from the entrance pupil of the condenser,
f' - the focal distance of the condenser,
d - the diameter of the light sensitive surface of the light detector,
c' - the maximal diameter of the scanning spot of the paper web.

The prior art disclosures are mainly oriented to the assessment of quality of paper specimens, and they do not analyze changes in the paper web density structure in the process of fabrication versus parameters of the machine.

As compared to the analogs, the proposed system forms the measuring information, which reflects reliably the parameters of the density structure of the paper web (a spatiotemporal spectrum of density non-uniformity of the paper web, a standard deviation of this non-uniformity, etc) and makes it possible to fully use this information for the adjustment and diagnostics of technical condition of papermaking machines and for the assessment of quality of paper produced.

For example, the use in the invention of specific optics of the illuminator and light detecting units enables to exclude the effect of paper web surface position fluctuations on the accuracy of measurements. This technical solution can be explained in the following way.

During photometric measurements of a moving paper web, its surface is illuminated by a spot beam, the power axis of which is normal to this surface. A scanning spot of assigned size c' is thereby formed on the surface. The parameters of the lens of the illuminator unit can be selected in such a way as to prevent the size c' from substantial changes during paper web plane fluctuations, that is to ensure the permanent position of the paper web surface within the range of the depth resolution of the illuminator image. As an example,. the lens can include an axicon [see B.L. Begunov et al., Theory of optical systems. Textbook for higher educational institutes. "Mashinostroenie" publishing house, 1981, p.88], which converts a point in the lens objects space into an axial line of a given length in the image space. When using a photo generator as an illuminator, the zone under consideration of depth resolution of the beam illuminating the paper web (a waist zone of the laser beam) may reach several centimeters. From the opposite side of the paper web, the scanning spot zone may be considered as a secondary illuminator. However, due to diffusing properties of paper [see A.S. Dubovik et al. Applied optics. Textbook for higher educational institutes. Moscow, "Nedra" publishing house, 1982, p. 140], the radiation of the secondary illuminator is distributed inside a hemisphere, that is inside the spatial angle of $2\pi$ steradian. Therefore, due to fluctuations of the paper web, changing the distance from the secondary illuminator considered, the signal from the light detector will be also changing, being inversely proportional to the square of the distance. Thus, a false component of the signal of the light detector will be formed, which, due to random nature of fluctuation of the position of the moving paper web, cannot be filtered out. To exclude the said error in laboratory specimens of equipment intended for analyses of the structure of paper by using optoelectronic methods, a sample of the paper web is pressed between two glass disks rotating at a constant speed as shown in the Russian patent No 1794247, class GO1 No 21/89 published in 1993. Evidently, in case of moving paper web such method is unacceptable.

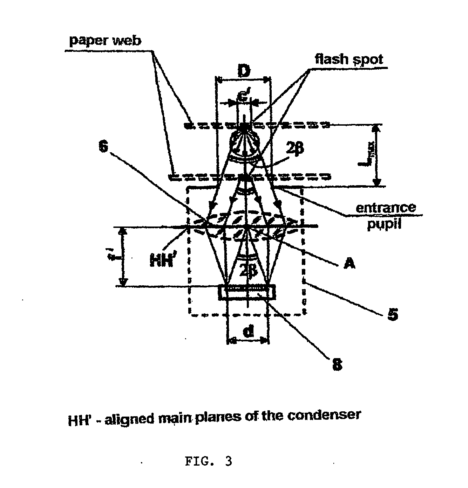

The following technical solution is proposed in this invention to exclude the impact of fluctuation of the paper web on accuracy of measurements. The optical part of the light detector unit (Fig. 3) is designed as a condenser with a light detector (photodiode) placed in its focal plane, the diameter D of the entrance pupil of the condenser, the diameter $d$ of the light sensitive surface of the light detector, the size $c'$ of scanning spot and the focal distance $f'$ of the condenser being correlated with the maximal permissible distance $L_{max}$ of the paper web from the entrance pupil of the condenser by the formula

$$L_{max} = \frac{D - c'}{d} \cdot f' \qquad (1)$$

The minimal distance $L_{min}$ is selected from structural considerations so as to secure possible fluctuations of the paper web plane within the range from $L_{min}$ to $L_{max}$, and the focusing plane of the light source lens is positioned at a distance of $\overline{L} = 0.5 \cdot (L_{max} + L_{min})$. The vision field angle $2\beta = 2 \cdot arctg \, d/2f'$ of the light detector unit determines the direction of light beams that fall on the entrance pupil of the condenser and are perceived by the light detector. It means that the beams that are inclined to the hairline of the light detector unit (for the hairline - see G.V.Pogarev. Adjustment of optical devices. Leningrad. "Mashinostroenie" publishing house, Leningrad branch, 1982, p.21-22) by an angle $\beta$ pass through the centre of the light detecting area and the nodal point A of the condenser (see Fig. 3). The beams with angles in excess of $\beta$ fail to fall onto the light detector. With such selection of the parameters of the light detector unit (see formula (1)), fluctuations of the paper web within the range $L \in (L_{min}, L_{max})$, i.e. from $L_{min}$ to $L_{max}$, will only cause changes of the condenser entrance pupil effective diameter.

$$D_{eff} = \frac{L \cdot d}{f'} + c' \qquad (2)$$

In this case, always cut out of the whole spatial angle equal to $2\pi$ steradian and used will only be the light flow, which is formed by light beams inclined to the hairline of the light detector unit at angles from 0 to $\beta$ and forming a spatial angle $\omega = 2n \cdot (1 - cos \, \beta)$, corresponding to a plane angle $\beta$. The selection of the condenser entrance pupil diameter

$$D = D_{eff \, max} = \frac{L_{max} \cdot d}{f'} + c', \qquad (3)$$

which meets formula (1), with a constant value of transmittance of the paper web and the value of distance $L$ changing from $L_{min}$ to $L_{max}$, ensures permanency of the light flow falling on the light detection area and hence - invariability of the light detector signal level.

Hence the technical solution claimed makes it possible to exclude the false component of the signal of the light detector, caused by possible fluctuation of the position of the plane of the moving paper web.

To exclude the impact of foreign flashes on the accuracy of measurements, a LED, or a photo generator, modulated by the current with a frequency employed as an information signal carrier frequency is used in the proposed invention.

The light sources in use have a narrow spectral radiation band, and they may be regarded as sources of monochromatic radiation. This circumstance makes it possible to select the radiation wave length corresponding to the spectral window of water transparency. In this way, the impact of water content in paper on the accuracy of measurements is excluded and structural properties and changes of thickness of paper only are analyzed.

Besides, a light filter, the spectral window of which corresponds to the wave length of the radiation source used, is introduced in the optical channel for the purpose of spectral selection of the signal.

Unlike the prototype, the method of extraction and processing of the signal of measuring information in the system proposed is based on analog-digital processing with final real-time computer-aided analysis, which enhances substantially the functional opportunities of the system from the standpoint of simultaneous calculation of a number of diagnostic parameters such as spatiotemporal spectrum of the signal, its mean square value, etc.

Hence, a conclusion can be made that the invention proposed provides the technical result, which includes increasing reliability of the measuring information obtained, ensuring address diagnostics of papermaking machines, as well as enhancing functional opportunities of the system by using software of the computer-aided analysis of the signal and the form of presentation of the results of the measurements.

The invention is believed to be new as no similar technical solutions with similar combination of distinctive features were found in available sources of information.

The invention is applicable in industry as it can be used for adjustment and diagnostics of technical condition of papermaking machines in pulp and paper industry.

BRIEF DESCRIPTION OF THE DRAWINGS

The invention proposed is explained by drawings, in which:

in Figure 1, a structural diagram of the first embodiment of the proposed optoelectronic system for adjustment and diagnostics of technical condition of papermaking machines is shown;
presented in Figure 2 is a structural diagram of the second embodiment of the proposed optoelectronic system for adjustment and diagnostics of technical condition of papermaking machines; and
in Figure 3, the diagram explaining the selection of parameters of the optical part of the light detecting unit is shown.

DESCRIPTION OF THE EMBODIMENTS OF THE INVENTION

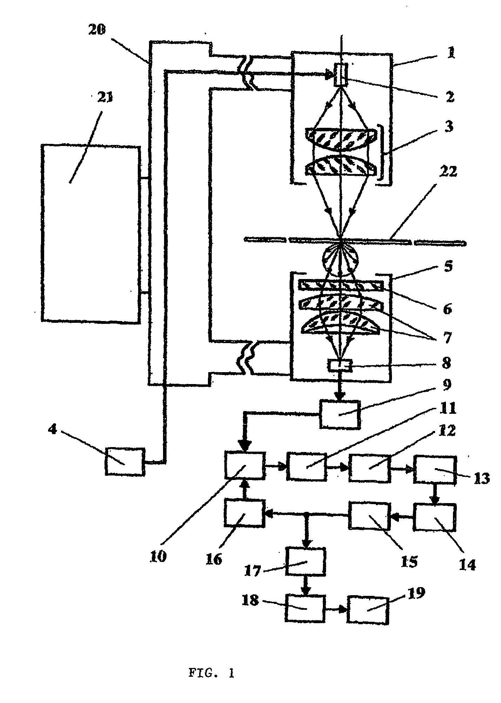

An optoelectronic system for adjustment and diagnostics of technical condition of papermaking machines in accordance with the first embodiment of the present invention (Figure 1) comprises an illuminator unit 1, which contains a light source 2 and a focusing optical system 3. The light source 2 (a LED or a photo generator (a semiconductor laser)) is powered from a carrier frequency current generator 4. The system also comprises a light detecting unit 5, which includes arranged successively in the direction of propagation of the beams in the same optical axis with the illuminator unit 1 a light filter 6 and a condenser 7, with a light detector 8 located in the focal plane of the condenser 7. An output of the light detector 8 is connected to an input of a pre-amplifier 9, an output of which is connected to a signal input of a digital potentiometer 10 series-connected via a band-pass filter 11, an amplifier 12, an amplitude detector13 and a low-pass filter 14 to a conditioning amplifier 15. An output of the amplifier 15 is connected to an analog input of a controller 16 and to an input of an analog-digital converter 17, and an output of controller 16 is connected to a control input of the digital potentiometer 10. An output of the analog-digital converter 17 is connected via an interface 18 to a personal computer 19. The system also includes a holder 20, intended for securing the illuminator unit 1, light detecting unit 5, and a travel gear 21. Figure number 22 denotes a paper web.

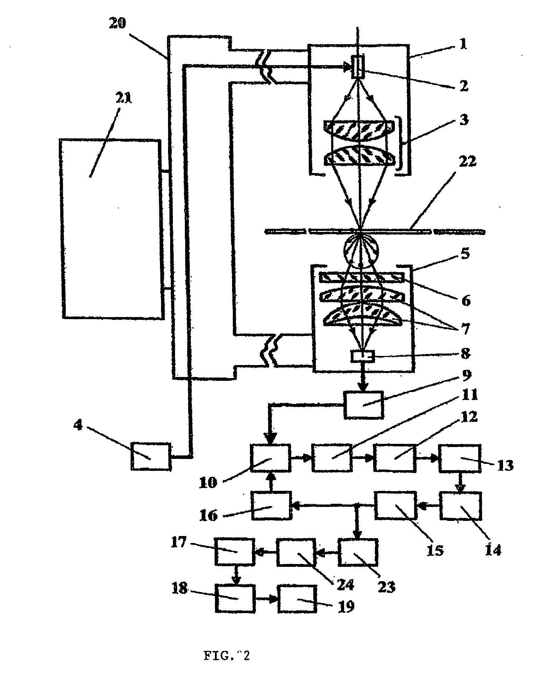

The optoelectronic system for adjustment and diagnostics of technical condition of papermaking machines in accordance with the second embodiment of the present invention (Figure 2) comprises, similar to the system shown in Figure 1, the illuminator unit 1 including the light source 2 and focusing optic system 3. The light source 2 (LED or photo generator) is powered from the carrier frequency current generator 4. The system also comprises the light detecting unit 5, which includes arranged in-series in the direction of propagation of the beams in the same optical axis with the illuminator unit 1 the light filter 6 and condenser 7, with the light detector 8 located in the focal plane of the condenser 7. The output of the light detector 8 is connected to the input of the pre-amplifier 9, the output of which is connected to the signal input of the digital potentiometer 10, connected in-series via the band-pass filter 11, amplifier 12, amplitude detector 13 and low-pass filter 14 to the conditioning amplifier 15, the output of the amplifier 15 is connected to the analog input of the controller 16; the output of the controller is connected to the control input of the digital potentiometer 10. The system, according to Figure 2, also contains the analog-digital converter 17, interface 18, personal computer 19, holder 20, travel gear 21, as well as additionally added an analog differential line driver 23 and a differential line receiver 24; the output of the conditioning amplifier 15 is additionally connected to an input of the analog differential line driver 23, and the output of the driver 23 is connected via the differential line receiver 24 to the input of the analog-digital converter 17. The output of analog digital converter 17 is connected via the interface 18 to the personal computer 19. Similar to Figure 1, figure number 22 denotes the paper web. The controller 16 can be designed based on IC of PIC12F630 type.

INDUSTRIAL APPLICABILITY

The system operates as follows. A signal comes from the light detector 8 to the signal input of the digital potentiometer 10 after having been amplified by the pre-amplifier 9. Due to the connection of the controller 16 to the control input of the potentiometer 10, the latter provides the amplification factor of the electronic path assigned by the controller 16. From the output of the potentiometer 10, the signal goes to the band-pass filter 11, which contributes to the formation of the frequency-response characteristic of the path with the extraction of the desired signal at a carrier frequency. Next, the signal is sent to the amplifier 12 and then to the amplitude detector 13, which extracts the envelope of the modulated signal. Further, the signal is transmitted to the low-pass filter 14, the cutoff frequency of which corresponds to the maximal frequency of the desired signal. From the output of the low-pass filter 14, the signal comes to the conditioning amplifier 15 that provides forming the signal changing within the full range of voltages of the power supply, in which capacity the carrier frequency current generator 4 is used. From the output of the conditioning amplifier 15, the signal arrives at the analog input of the controller 16. When power supply of the system is activated and the paper web 22 is available, the controller starts controlling the digital potentiometer 10 in such a way as to secure the voltage at the output of the conditioning amplifier 15 to be a half of the power supply voltage. This status achieved is preserved during the whole session of operation with this paper web 22. From the output of the conditioning amplifierЯ 15, the signal also comes to the input of the analog-digital converter 17, and from the output of the latter it is transmitted via the interface 18 to a port of the personal computer 19.

To provide high speed of transmission of information it is expedient to use a USB port of the computer and a relevant interface.

In this case a special-purpose software enables to calculate and represent graphically in all but real time the measuring signal, the amplitude of which is proportional to the density non-uniformity of the paper web, as well as statistical parameters thereof reduced to the dimensionality required, e.g. spatiotemporal spectrum, and others coordinated by known diagnostic procedures with parameters of adjustment of the papermaking machine.

However in this case the length of the cable that connects the computer with the optoelectronic part of the system is limited by a value of about 1,5 m.

In the event that the personal computer 19 is located at a considerable distance from the papermaking machine, the signal from the output of the conditioning amplifier 15 is applied to the analog differential line driver 23, which converts the signal from a unipolar to a differential form to improve noise-immunity of the system. Next, the signal comes to the differential line receiver 24, which restores the differential signal converting it back to the unipolar form. Then the signal is transmitted to the analog-digital converter 17 and via the interface 18 arrives at the port of the personal computer 19.

Thus, the proposed optoelectronic system for adjustment and diagnostics of technical condition of papermaking machines makes it possible to improve reliability of measuring information and based on the analysis of this information to optimally conduct adjustment and address diagnostics of the papermaking machine.

**Claims**

1.  An optoelectronic system for adjustment and diagnostics of technical condition of papermaking machines, comprising an illuminator unit with a light source with a focusing optic system, a light detecting unit with a light detector, a pre-amplifier, **characterized by** that a LED, or a photo generator, is used as the light source, the light source being powered by a carrier frequency current generator, which provides relevant modulation of radiation of the light source, the light detecting unit further comprises a light filter and a condenser arranged in-series in the direction of propagation of the beams and aligned with an optical axis of the illuminator unit, the light detector being located in a focal plane of the condenser, an output of the light detector is connected to an input of the pre-amplifier, an output of the pre-amplifier is connected to a signal input of a digital potentiometer whose output is coupled through series-connected a band-pass filter, an amplifier, an amplitude detector, and a low-pass filter with a conditioning amplifier, an output of the conditioning amplifier is connected to an analog input of a controller and to an input of an analog-digital converter, an output of the controller is connected to a control input of the digital potentiometer, and an output of the analog-digital converter is connected via an interface to a personal computer, the diameter of the entrance pupil of the condenser being calculated by the formula:

$$D = \frac{L_{max}}{f'} \bullet d + c'$$

where:

> $D$ - the diameter of the entrance pupil of the condenser,
> $L_{max}$ - the maximal permissible distance of the paper web from the entrance pupil of the condenser,
> $f'$ - the focal distance of the condenser,

*d* - the diameter of the light sensitive surface of the light detector, and
*c'* - the maximal diameter of the scanning spot of the paper web.

2. An optoelectronic system for adjustment and diagnostics of technical condition of papermaking machines, comprising an illuminator unit with a light source with a focusing optic system, a light detecting unit with a light detector, a pre-amplifier, **characterized by** that a LED, or a photo generator, is used as the light source, the light source being powered by a carrier frequency current generator, which provides relevant modulation of radiation of the light source, the light detecting unit further comprises a light filter and a condenser arranged in-series in the direction of propagation of the beams and aligned with an optical axis of the illuminator unit, the light detector being located in a focal plane of the condenser, an output of the light detector is connected to an input of the pre-amplifier, an output of the pre-amplifier is connected to a signal input of a digital potentiometer whose output is coupled through series-connected a band-pass filter, an amplifier, an amplitude detector, and a low-pass filter with a conditioning amplifier, an output of the conditioning amplifier is connected to an analog input of a controller and to an input of an analog differential line driver; an output of the controller is connected to a control input of the digital potentiometer, and an output of the analog differential line driver is connected via a differential line receiver to an input of a analog-digital converter, an output of which is connected via an interface to a personal computer, the diameter of the entrance pupil of the condenser being calculated by the formula:

$$D = \frac{L_{max}}{f'} \bullet d + c'$$

where:

D - the diameter the entrance pupil of the condenser,
$L_{max}$ - the maximal permissible distance of the paper web from the entrance pupil of the condenser,
f' - the focal distance of the condenser,
d - the diameter of the light sensitive surface of the light detector,
c' - the maximal diameter of the scanning spot of the paper web.

FIG. 1

FIG. 2

HH' - aligned main planes of the condenser

FIG. 3

## INTERNATIONAL SEARCH REPORT

| International application No. |
| --- |
| PCT/RU2006/000436 |

A.    CLASSIFICATION OF SUBJECT MATTER     *D21F 7/06  (2006.01)*
*G01N 21/89  (2006.01)*

According to International Patent Classification (IPC) or to both national classification and IPC

B.    FIELDS SEARCHED

Minimum documentation searched (classification system followed by classification symbols)

D21F 7/00, 7/04, 7/06, G01N 21/00, 21/84, 21/86, 21/89, D21G 9/00

Documentation searched other than minimum documentation to the extent that such documents are included in the fields searched

Electronic data base consulted during the international search (name of data base and, where practicable, search terms used)

C.    DOCUMENTS CONSIDERED TO BE RELEVANT

| Category* | Citation of document, with indication, where appropriate, of the relevant passages | Relevant to claim No. |
| --- | --- | --- |
| A | RU 195868 A (UKRAINSKY NAUCHNO-ISSLEDOVATELSKY INSTITUT TSELLJULOZNOI I BUMAZHNOI PROMYSHLENNOSTI) 11.07.1967, column 1, line 21 - column 2, line 19, the figure | 1-2 |
| A | RU 896132 A (TSENTRALNY NAUCHNO-ISSLEDOVATELSKY INSTITUT BUMAGI) 07.01.1982, column 2, lines 17-22 | 1-2 |
| A | RU 2224994 C2 (FILIP MORRIS PRODUCTS INC.) 27.02.2004, page 12, column 1, lines 52-66 | 1-2 |
| A | EP 0401188 A2 (VALMET PAPER MACHINERY INC.) 05.12.1990 | 1-2 |
| A | US 4845374 A (R.J. REYNOLDS TOBACCO COMPANY) 04.07.1989, the abstract, figure 1 | 1-2 |
| A | GB 2081891 A (THE WIGGINS TEAPE GROUP LIMITED) 24.02.1982, the abstract, figure 1 | 1-2 |

☐   Further documents are listed in the continuation of Box C.          ☐   See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 December 2006 (08.12.2006) | 28 December 2006 (28.12.2006) |
| Name and mailing address of the ISA/    **RU** | Authorized officer |
| Facsimile No. | Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 896132 A
- US 195868 A
- RU 1794247

### Non-patent literature cited in the description

- Theory of optical systems. **B.L. BEGUNOV et al.** Textbook for higher educational institutes. Mashinostroenie, 1981, 88
- Applied optics. **A.S. DUBOVIK et al.** Textbook for higher educational institutes. Moscow. Nedra, 1982, 140
- Adjustment of optical devices. **G.V.POGAREV.** Leningrad. Mashinostroenie, 1982, 21-22